# EUROPEAN PATENT APPLICATION

(11) **EP 2 216 337 A1**
(43) Date of publication of application: **11.08.2010**
(21) Application number: 08858948.6
(22) Date of filing: 26.11.2008
(51) Int. Cl.: C07H 15/04, C07H 1/00, C07H 5/02

(54) **METHOD FOR PRODUCING 4-DEOXY-4-FLUORO-D-GLUCOSE DERIVATIVE**

(30) Priority: 12.12.2007 JP 2007320913
(71) Applicant: Central Glass Company, Limited, Yamaguchi 755-0001 (JP)
(72) Inventor: ISHII, Akihiro, Kawagoe-shi Saitama 350-1151 (JP); YASUMOTO, Manabu, Kawagoe-shi Saitama 350-1151 (JP); OOTSUKA, Takashi, Kawagoe-shi Saitama 350-1151 (JP); NIGORIKAWA, Yasuko, Kawagoe-shi Saitama 350-1151 (JP)
(74) Representative: Manitz, Finsterwald & Partner GbR
(86) International application number: PCT/JP2008/071380
(87) International publication number: WO 2009/075186

(57) **Abstract**

There is disclosed a method for producing a 4-deoxy-4-fluoro-D-glucose derivative by reacting a D-galactose derivative with either sulfuryl fluoride (SO₂F₂), trifluoromethanesulfonyl fluoride (CF₃SO₂F) or perfluorobutanesulfonyl fluoride (C₄F₉SO₂F) in the presence of an organic base or in the presence of an organic base and a salt or complex of an organic base and hydrogen fluoride.

## Description

### Technical Field

The present invention relates to an industrial production method (suitable for large-scale production) of a 4-deoxy-4-fluoro-D-glucose derivative, which is an important intermediate for pharmaceutical and agrichemical products, notably drugs for diabetes.

### Background Art

4-Deoxy-4-fluoro-D-glucose derivatives are important intermediates for pharmaceutical and agrichemical products, notably drugs for diabetes. The conventional production processes of these derivatives use (diethylamino)sulfur trifluoride (DAST) or [bis(2-methoxyethyl)amino]sulfur trifluoride (BAST). (See Non-Patent Document 1 and Patent Document 1.) However, the above dehydroxyfluorination agents are expensive; and the DAST has a danger of explosion. The conventional production processes are thus limited to small-scale production uses and cannot be applied to industrial productions. Further, the conventional production processes suitably use methylene chloride, which is limited in industrial use, as a reaction solvent and shows a medium level of yield. The industrial application of the conventional production processes is also interfered with by these factors.

For the above reasons, it has been impossible to produce 4-deoxy-4-fluoro-D-glucose derivatives on an industrial scale.

On the other hand, the present applicant has disclosed a dehydroxyfluorination reaction process that uses sulfuryl fluoride (SO₂F₂) in combination with an organic base (optionally in the presence of "a salt or complex of an organic base and hydrogen fluoride"). (See Patent Document 2.)
Patent Document 1: International Application Publication No. WO 2004/052903 (Japanese Translation of International Application No. 2006-510644)
Patent Document 2: International Application Publication No. WO 2006/098444 (Japanese Laid-Open Patent Publication No. 2006-290870)
Non-Patent Document 1: Journal of Organic Chemistry (U.S.), 1983, Vol. 48, p. 393-395

### Disclosure of the Invention

It is an object of the present invention to provide an industrial production method of a 4-deoxy-4-fluoro-D-glucose derivative. As a necessary solution to the conventional problems, it is of most importance to use a dehyroxyfluorination agent that is available at low cost and has no danger of explosion. It is also important to find a technique for high-yield production of the target compound without the need to use methylene chloride that is limited in industrial use.

As a result of extensive researches made in view of the above tasks, the present inventors have found that the dehydroxyfluorination reaction process of Patent Document 2, which uses sulfuryl fluoride in combination with an organic base (optionally in the presence of "a salt or complex of an organic base and hydrogen fluoride"), is particularly suitably applicable to a D-galactose derivative, i.e., a raw material of the present invention, such that the dehydroxyfluorination reaction of the D-galactose derivative can proceed favorably to produce a 4-deoxy-4-fluoro-D-glucose derivative as a target compound with high yield. The present inventors have further found that: although the above dehydroxyfluorination reaction process introduces a fluorine atom while inverting the configuration of a 4-position hydroxyl group of the D-galactose derivative at a very high rate; and that there occurs less competitive by-product such as olefin during the fluorine substitution subsequent to the fluorosulfonylation. It is therefore possible in the present invention to produce the 4-deoxy-4-fluoro-D-glucose derivative even by a simple purification operation with high purity and with almost no impurity by-product that is difficult to separate from the target compound.

In the case of using sulfuryl fluoride particularly suitable as a dehydroxyfluorination agent, the post-reaction solution stoichiometrically contains "a salt of fluorosulfuric acid and organic base". If such a salt remains in the crude product or final product of the target compound, there arises not only a problem that the fluorosulfuric acid itself exerts an unfavorable toxic effect but also other problems such that: the fluorosulfuric acid acts as an acid catalyst to cause elimination of a hydroxyl protecting group; the recovery rate of the target compound by recrystallization purification becomes decreased; and the fluorine ion concentration becomes increased due to decomposition over time. It is thus important to remove the salt efficiently by post-treatment operation. The present inventors have found that the operation of washing an organic layer containing the target compound and the salt with water or an aqueous alkaline solution allows the salt, which is highly soluble in water, to be efficiently and selectively extracted into an aqueous layer and have verified that the water washing of the organic layer can be applied as an effective technique to remove the salt.

The present inventors have also found that the above salt removal technique can result in a deterioration in the efficiency of removing "the salt of fluorosulfuric acid and organic base" in the case of reducing the amount of the water or aqueous alkaline solution used in view of industrialization. (See Example 1.) As both of the raw material of the invention, i.e., D-galactose derivative and the target compound of the present invention, i.e., 4-deoxy-4-fluoro-D-glucose derivative have low solubility in organic solvents, there is a need to use a large amount of organic solvent during the reaction and during the post-treatment operation. It is more difficult to efficiently remove the salt from such a diluted organic layer (with the use of a limited amount of water or aqueous alkaline solution). The waste water amount cannot be reduced even by the removal technique of Patent Document 2.

The present inventors have found, based on the fact that the solubility of the target 4-deoxy-4-fluoro-D-glucose derivative in the organic solvent is low, that the addition of water to the post-reaction solution favorably leads to the formation of a crystalline precipitate of the target compound so that "the salt of fluorosulfuric acid and organic base" can be concentrated in the filtrate after the filtration with almost none of the salt contained in the recovered crystalline precipitate. This post-treatment operation enables significant reductions in waste water amount and waste organic solvent amount and contributes to very good operability and high productivity through the effective use of the physical properties of the target compound. (See Example 2.)

In the present invention, the same reactivity can be obtained in the case of using trifluoromethanesulfonyl fluoride (CF₃SO₂F) or perfluorobutanesulfonyl fluoride (C₄F₉SO₂F) as the dehydroxyfluorination agent in place of sulfuryl fluoride. In this case, "a salt of perfluoroalkanesulfonic acid and organic base" is formed stoichiometrically as a by-product of the reaction. The lipid solubility of "the salt of perfluoroalkanesulfonic acid and organic base" increases with the carbon number of the perfluoroalkyl group.
The sulfuryl fluoride, which forms the highest water-soluble "salt of fluorosulfuric acid and organic base" as the reaction by-product, is thus particularly suitable to make the most effective use of the merit of the post-treatment operation in the present invention.

As mentioned above, the present inventors have found the particularly effective techniques for industrial production of the 4-deoxy-4-fluoro-D-glucose derivative. The present invention is made based on these findings.

Namely, the present invention provides first to eighth methods for industrial production of a 4-deoxy-4-fluoro-D-glucose derivative.

There is provided according to the present invention a method (first method) for producing a 4-deoxy-4-fluoro-D-glucose derivative of the formula [2], comprising: performing a reaction of a D-galactose derivative of the formula [1] with either sulfuryl fluoride (SO₂F₂), trifluoromethanesulfonyl fluoride fluoride (CF₃SO₂F) or perfluorobutanesulfonyl fluoride (C₄F₉SO₂F) in the presence of an organic base 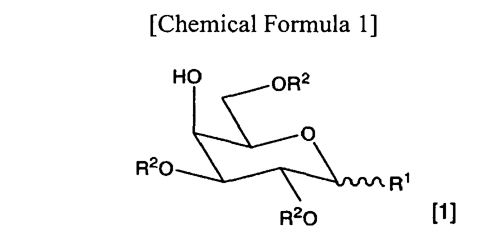 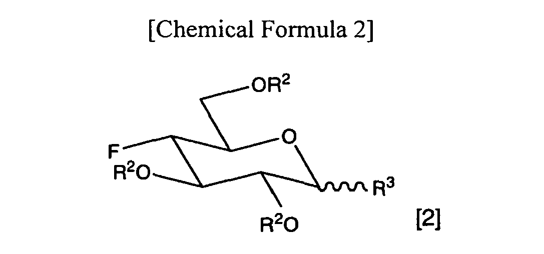 where R¹ represents a hydroxyl group, a lower alkoxy group, a lower acyloxy group or a halogen atom; R² independently represents a hydroxyl protecting group; R³ represents a lower alkoxy group, a lower acyloxy group or a halogen atom; and the wavy line represents the presence of either or both of α-anomer configuration and β-anomer configuration.

The first method may be the method (second method) for producing the 4-deoxy-4-fluoro-D-glucose derivative, in which the reaction is performed in the additional presence of a salt or complex of an organic base and hydrogen fluoride in the reaction system.

There is also provided according to the present invention a method (third method) for producing a 4-deoxy-4-fluoro-α-D-glucopyranoside derivative of the formula [4], comprising: performing a reaction of an α-D-galactopyranoside derivative of the formula [3] with either sulfuryl fluoride (SO₂F₂) or trifluoromethanesulfonyl fluoride (CF₃SO₂F) in the presence of an organic base, thereby obtaining a post-reaction solution containing the 4-deoxy-4-fluoro-α-D-glucopyranoside derivative as a target compound; and forming and recovering a crystalline precipitate of the target compound with the addition of water to the post-reaction solution 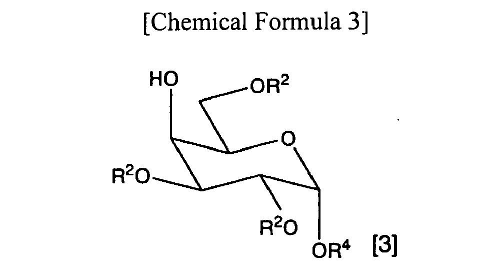 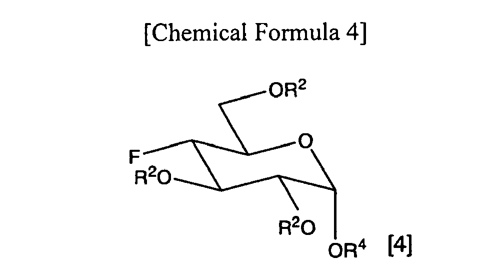 where R² independently represents a hydroxyl protecting group; and R⁴ represents a lower alkyl group.

The third method may be the method (fourth method) for producing the 4-deoxy-4-fluoro-α-D-glucopyranoside derivative, in which the reaction is performed in the additional presence of a salt or complex of an organic base and hydrogen fluoride in the reaction system.

The third or fourth method may be the method (fifth method) for producing the 4-deoxy-4-fluoro-α-D-glucopyranoside derivative, in which a water-miscible organic solvent is used as a solvent of the reaction; and an amount of the water added to the post-reaction solution to form and recover the crystalline precipitate of the target compound is one-third to three times an amount of the solvent of the reaction.

There is further provided according to the present invention a method (sixth method) for producing methyl 2,3,6-tri-O-benzoyl-4-deoxy-4-fluoro-α-D-glucopyranoside of the formula [6], comprising: performing a reaction of methyl 2,3,6-tri-O-benzoyl-α-D-galactopyranoside of the formula [5] with sulfuryl fluoride (SO₂F₂) in the presence of triethylamine, thereby obtaining a post-reaction solution containing the methyl 2,3,6-tri-O-benzoyl-4-deoxy-4-fluoro-α-D-glucopyranoside as a target compound; and forming and recovering a crystalline precipitate of the target compound with the addition of water to the post-reaction solution 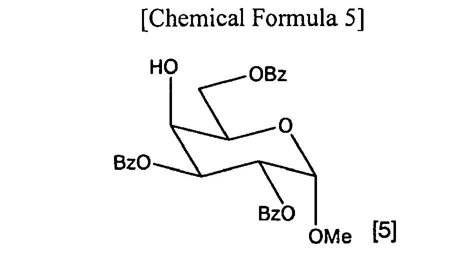 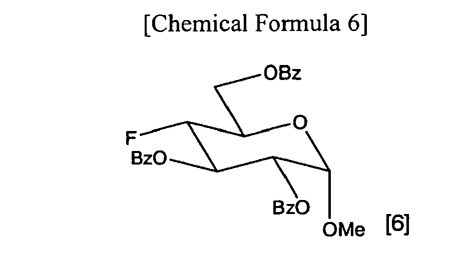 where Me represents a methyl group; and Bz represents a benzoyl group.

The sixth method may be the method (seventh method) for producing the methyl 2,3,6-tri-O-benzoyl-4-deoxy-4-fluoro-α-D-glucopyranoside, in which the reaction is performed in the additional presence of a salt or complex of triethylamine and hydrogen fluoride.

The sixth or seventh method may be the method (eighth method) for producing the methyl 2,3,6-tri-O-benzoyl-4-deoxy-4-fluoro-α-D-glucopyranoside, in which a water-miscible organic solvent is used as a solvent of the reaction; and an amount of the water added to the post-reaction solution to form and recover the crystalline precipitate of the target compound is one-third to three times an amount of the solvent of the reaction.

### Detailed Description

The advantages of the present invention over the earlier technologies will be explained below.

The present invention is advantageous over Non-Patent Document 1 and Patent Document 1, in that the production method of the present invention uses as a dehydroxyfluorination agent either of sulfuryl fluoride, trifluoromethanesulfonyl fluoride and perfluorobutanesulfonyl fluoride, each of which is available at low cost and has no danger of explosion. The sulfuryl fluoride, which is particularly suitable as the dehydroxyfluorination agent in the present invention, is widely used as a fumigant and is easily available in large quantity. It is also advantageous in that the production method of the present invention can favorably use various reaction solvents and avoid the use of methylene chloride and can provide a much higher yield of the target compound than those of the earlier technologies.

The present invention is advantageously based on the finding that the dehydroxyfluorination reaction process of Patent Document 2 is particularly suitably applicable to the raw material of the present invention, i.e., D-galactose derivative. It is further advantageous, in that the post-treatment operation of the present invention enables significant reductions in waste water and waste organic solvent and allows good operability and high productivity.

It is accordingly possible in the present invention to produce the 4-deoxy-4-fluoro-D-glucose derivative with high purity and yield and to solve all of the conventional problems so that the production method of the present invention is industrially easily realizable.

A production method of the 4-deoxy-4-fluoro-D-glucose derivative according to the present invention will be described in more detail below.

The production method of the present invention involves a reaction of a D-galactose derivative of the formula [1] with sulfuryl fluoride (trifluoromethanesulfonyl fluoride, or perfluorobutanesulfonyl fluoride) in the presence of an organic base or in the presence of an organic acid and "a salt or complex of an organic base and hydrogen fluoride". This makes it possible to carry out fluorosulfonylation (trifluoromethanesulfonylation or perfluorobutanesulfonylation) and fluorine substitution continuously in one reactor without isolating a reaction intermediate such as fluorosulfuric ester (trifluoromethanesulfonic ester or perfluorobutanesulfonic ester). The stereochemical configuration of a hydroxyl group of the reactant is maintained in the fluorosulfonylation (trifluoromethanesulfonylation or perfluorobutanesulfonylation) and is inverted in the subsequent fluorine substitution. The D-galactose derivative of the formula [1] can be thus converted to the 4-deoxy-4-fluoro-D-glucose devivative of the formula [2].

Examples of R¹ of the D-galactose derivative of the formula [1] are a hydroxyl group, C₁-C₆ straight-chain or branched alkoxy groups, C₁-C₆ straight-chain or branched acyloxy groups and halogen atoms such as fluorine, chlorine and bromine. Among others, preferred are C₁-C₆ straight-chain or branched alkoxy group. A methoxy group is particularly preferred. When R¹ is a substituent group other than hydroxyl, the substituent group R¹ does not change before and after the reaction (which means that R¹ is the same as R³ of the 4-deoxy-4-fluoro-D-glucose derivative of the formula [2]).

Examples of R² of the D-galactose derivative of the formula [1] are hydroxyl protecting groups such as acyl groups e.g. an acetyl group and a benzoyl group and aralkyl groups e.g. a benzyl group. Among others, acyl groups such as acetyl and benzoyl are preferred. Particularly preferred is benzoyl. The hydroxyl protecting groups can be selected independently. There are a case where the three groups are different from one another, a case where two of the three groups are the same and the other one is different, and a case where the three groups are the same. It is preferable that two of the three groups are the same and the other one is different, or the three groups are the same. It is particularly preferable that the three groups are the same. The substituent group R² does not change before and after the reaction.

The wavy line indicates that the D-galactose derivative of the formula [1] has either or both of α-anomer configuration and β-anomer configuration. The anomer configuration of the D-galactose derivative can be selected depending on the anomer configuration of the target 4-deoxy-4-fluoro-D-glucose derivative of the formula [2]. When R¹ is a substituent group other than hydroxyl, the anomer configuration is maintained before and after the reaction. When R¹ is a hydroxyl group, the ratio of α-anomer configuration and β-anomer configuration may be changed by replacement of the hydroxyl group with a fluorine atom.

When R³ of the 4-deoxy-4-fluoro-D-glucose derivative of the formula [2] is an fluorine atom, it is conceivable to select a hydroxyl group as R¹ of the D-galactose derivative of the formula [1] and react and replace the hydroxyl group with the fluorine atom.

Examples of R⁴ of the α-D-galactopyranoside derivative of the formula [3] are C₁-C₆ straight-chain or branched alkyl groups.

The D-galactose derivative of the formula [1] can be prepared with reference to Journal of Organic Chemistry (U.S.), 1965, Vol. 30, p. 2312-2317 and the like. The α-D-galactopyranoside derivative of the formula [3] is relatively easy to produce and is thus used suitably as the raw material of the present invention. Further, the methyl 2,3,6-tri-O-benzoyl-α-D-galactopyranoside of the formula [5] is commercially available and easy to get in large quantity and is thus used particularly suitably as the raw material of the present invention.

Either of sulfuryl fluoride (SO₂F₂), trifluoromethanesulfonyl fluoride (CF₃SO₂F) and perfluorobutanesulfonyl fluoride (C₄F₉SO₂F) is used as the dehydroxyfluorination agent. Among others, sulfuryl fluoride and trifluoromethanesulfonyl fluoride are preferred. Particularly preferred is sulfuryl fluoride. The sulfuryl fluoride has high fluorine atom economy and can be treated into fluorite (CaF₂) as a final waste. Although the sulfuryl fluoride has two reaction sites, it has been found that, even when the D-galactose derivative of the formula [1] is used as the raw material of the present invention and reacted with the sulfuryl fluoride, the fluorine substitution can be performed favorably to go through a desired fluorosulfuric ester and form almost no disubstituted compound such as sulfuric diester by adoption of the appropriate reaction conditions. (See Scheme 1.)

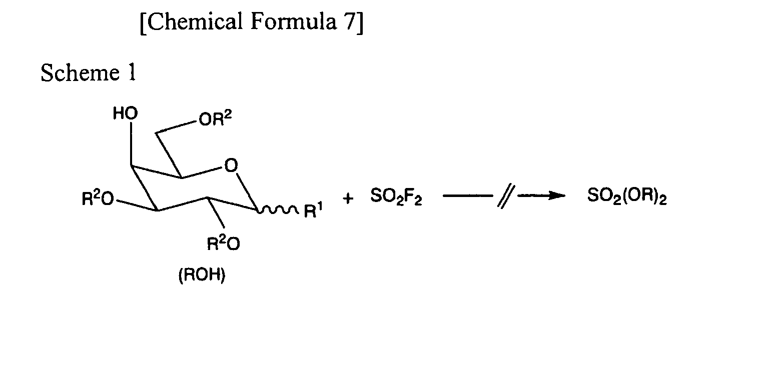

The amount of the dehydroxyfluorination agent used is not particularly limited. It suffices to use 1 mol or more of the dehydroxylfluorination agent per 1 mole of the D-galactose derivative of the formula [1]. The amount of the dehydroxyfluorination agent used is generally preferably in the range of 1 to 10 moles, more preferably 1 to 5 moles, per 1 mole of the D-galactose derivative of the formula [1]. (Although there is no problem in using the dehyroxyfluorination agent excessively as in Example 4, it is not economically favorable to use such an excessive amount of dehyroxyfluorination agent.)

Examples of the organic base are trimethylamine, triethylamine, diisopropylethylamine, tri-n-propylamine, tri-n-butylamine, pyridine, 2,3-lutidine, 2,4-lutidine, 2,5-lutidine, 2,6-lutidine, 3,4-lutidine, 3,5-lutidine, 2,3,4-collidine, 2,4,5-collidine, 2,5,6-collidine, 2,4,6-collidine, 3,4,5-collidine, 3,5,6-collidine, N,N-dimethylcyclohexylamine (DMCHA), 1,8-diazabicyclo[5.4.0]undece-7-ene (DBU) and dimethylaminopyridine (DMAP). Among others, triethylamine, diisopropylethylamine, tri-n-propylamine, tri-n-butylamine, pyridine, 2,3-lutidine, 2,4-lutidine, 2,6-lutidine, 3,4-lutidine, 3,5-lutidine, 2,4,6-collidine and 3,5,6-collidine are preferred. Particularly preferred is triethylamine.

The amount of the organic base used is not particularly limited. It suffices to use 1 mol or more of the organic base per 1 mole of the D-galactose derivative of the formula [1]. The amount of the organic base used is generally preferably in the range of 1 to 20 moles, more preferably 1 to 10 moles, per 1 mole of the D-galactose derivative of the formula [1].

"The salt or complex of the organic base and hydrogen fluoride" used in the second, fourth, fifth, seventh and eighth methods will be explained later in detail.

In the present invention, the D-galactose derivative of the formula [1] is converted to a fluorosulfuric ester with the use of e.g. sulfuryl fluoride and triethylamine; and "a salt of triethylamine and hydrogen fluoride", which is formed stoichiometrically as a by-product of the fluorosulfonylation in the reaction system, is used effectively as a fluorine source in the fluorine substitution as indicated in Scheme 2. Further, it has been found that the fluorosulfonylation can be performed in the presence of e.g. "triethylamine tris(hydrogen fluoride) complex" as indicated in Scheme 3 to produce the 4-deoxy-4-fluoro-D-glucose derivative of the formula [2] with higher yield and selectivity in the process of Scheme 3 than in the process of Scheme 2.

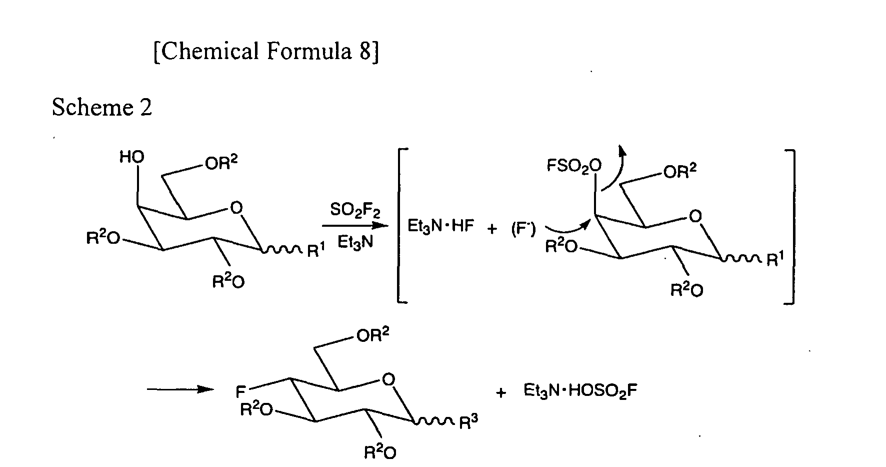

Case of using SO₂F₂ (1 equivalent) as dehydroxyfluorination agent and triethylamine (1 equivalent) as organic base

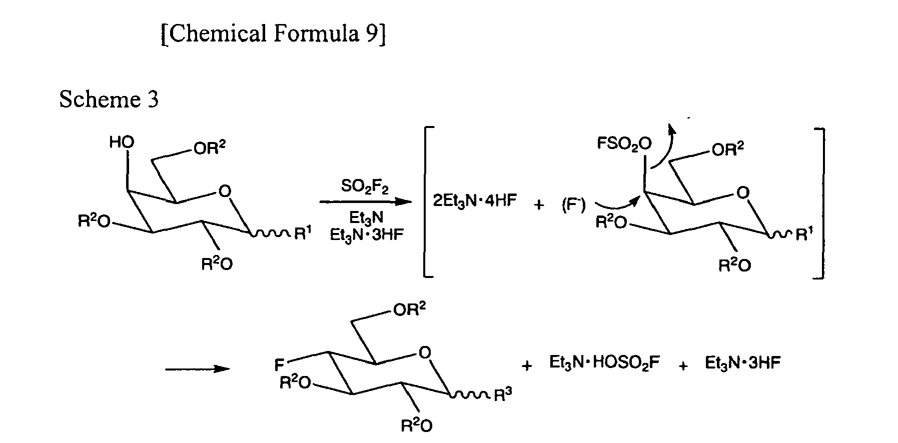

Case of using SO₂F₂ (1 equivalent) as dehydroxyfluorination agent, triethylamine (1 equivalent) as organic base and triethylamine tris(hydrogen fluoride) complex (1 equivalent) as "salt or base of organic base and hydrogen fluoride"

Examples of the organic base used in "the salt or complex of the organic base and hydrogen fluoride" are trimethylamine, triethylamine, diisopropylethylamine, tri-n-propylamine, tri-n-butylamine, pyridine, 2,3-lutidine, 2,4-lutidine, 2,5-lutidine, 2,6-lutidine, 3,4-lutidine, 3,5-lutidine, 2,3,4-collidine, 2,4,5-collidine, 2,5,6-collidine, 2,4,6-collidine, 3,4,5-collidine, 3,5,6-collidine, N,N-dimethylcyclohexylamine (DMCHA), 1,8-diazabicyclo[5.4.0]undece-7-ene (DBU) and dimethylaminopyridine (DMAP). Among others, triethylamine, diisopropylethylamine, tri-n-propylamine, tri-n-butylamine, pyridine, 2,3-lutidine, 2,4-lutidine, 2,6-lutidine, 3,4-lutidine, 3,5-lutidine, 2,4,6-collidine and 3,5,6-collidine are preferred. Particularly preferred is triethylamine.

The mole ratio of the organic base and hydrogen fluoride in "the salt or complex of the organic base and hydrogen fluoride" ranges from 100:1 1 to 1:100, generally preferably 50:1 to 1:50, more preferably 25:1 to 1:25. It is particularly convenient to use "a complex of 1 mole of triethylamine and 3 moles of hydrogen fluoride" or "a complex of 30% or less (10 mol% or less) of pyridine and 70% or less (90 mol% or less) of hydrogen fluoride", each of which is available from Aldrich (Aldrich 2007-2008 General Catalogue).

The amount of "the salt or complex of the organic base and hydrogen fluoride" used is not particularly limited. It suffices to use 0.3 mol or more of the salt or complex in terms of fluorine anion (F⁻) per 1 mole of the D-galactose derivative of the formula [1]. The amount of the salt or complex used is generally preferably in the range of of 0.5 to 50 moles, more preferably 0.7 to 25 moles, in terms of fluorine anion (F⁻) per 1 mole of the D-galactose derivative of the formula [1].

Examples of the reaction solvent are: aliphatic hydrocarbon solvents such as n-hexane, cyclohexane and n-heptane; aromatic hydrocarbon solvents such as benzene, toluene, xylene and mesitylene; halogenated hydrocarbon solvents such as methylene chloride, chloroform and 1,2-dichloroethane; ether solvents such as diethyl ether, tetrahydrofuran and tert-butyl methyl ether; ester solvents such as ethyl acetate and n-butyl acetate; amide solvents such as N,N-dimethylformamide, N,N-dimethylacetamide, N-methyl-pyrrolidone and 1,3-dimethyl-2-imidazolidinone; nitrile solvents such as acetonitrile and propionitrile; and dimethyl sulfoxide. Among others, tetrahydrofuran, N,N-dimethylformamide, N,N-dimethylacetamide, 1,3-dimethyl-2-imidazolidinone, acetonitrile, propionitrile and dimethyl sulfoxide are preferred. Particularly preferred are tetrahydrofuran, N,N-dimethylformamide and acetonitrile. These reaction solvents can be used solely or in any combination thereof.

The amount of the reaction solvent used is not particularly limited. It suffices to use 0.1 L (liter) or more of the reaction solvent per 1 mole of the D-galactose derivative of the formula [1]. The amount of the reaction solvent used is generally preferably in the range of 0.1 to 20 L, more preferably 0.1 to 10 L, per 1 mole the D-galactose derivative of the formula [1]. In the present invention, the reaction can be initiated in a state where a part of the raw material remains undissolved.

There is no particular limit on the reaction temperature. It suffices to conduct the reaction in the temperature range of -100 to +100°C. The reaction temperature is generally preferably in the range of -80 to +80°C, more preferably -60 to +60°C. Under the reaction temperature condition not lower than the boiling point of the dehydroxyfluorination agent (e.g. the boiling point (-49.7°C) of sulfuryl fluoride), the reaction can be conducted using a pressure-proof reaction vessel.

There is no particular limit on the pressure condition. It suffices to conduct the reaction in the pressure range of atmospheric pressure to 2 MPa. The pressure condition is generally preferably in the range of atmospheric pressure to 1.5 MPa, more preferably atmospheric pressure to 1 MPa. It is thus preferable to conduct the reaction with the use of the pressure-proof reaction vessel that is made of a stainless steel (SUS) material, a glass (glass-lined) material or the like.

There is no particular limit on the reaction time. It suffices to conduct the reaction in the range of 0.1 to 72 hours. The reaction time depends on the raw material and the reaction conditions. It is preferable to determine the time at which the raw material has almost disappeared as the end of the reaction while monitoring the progress of the reaction by any analytical means such as gas chromatography, liquid chromatography or NMR.

There is also no particular limit on the post-treatment operation. In general, the post-treatment operation can be performed by diluting the post-reaction solution with an organic solvent (such as toluene, xylene, tert-butyl methyl ether, ethyl acetate or the like), washing the diluted post-reaction solution with water or an aqueous solution containing an inorganic base of an alkaline metal (such as sodium hydrogencarbonate, potassium hydrogencarbonate, sodium carbonate, potassium carbonate or the like) (as in the conventional technique of removing "a salt of an organic base and fluorosulfuric acid"), and then, concentrating the recovered organic layer to obtain a crude product of the 4-deoxy-4-fluoro-D-glucose derivative of the formula [2].

One of the important features of the present invention is that the post-treatment operation is performed by forming a crystalline precipitate of the target compound with the addition of water to the post-reaction solution and recovering the formed crystalline precipitate from the post-reaction solution. This makes it possible to efficiently concentrate "the salt of the fluorosulfuric acid (or perfluoroalkanesulfonic acid) and organic base" into the filtrate after the filtration and enables high-yield recovery of the crude crystalline product with almost no salt. In order to maximize the merit of the post-treatment operation of the present invention, it is preferable to use N,N-dimethylformamide or acetonitrile of particularly high water miscibility as the reaction solvent and control the amount of the reaction solvent to 0.5 to 5 L per 1 mole of the D-galactose derivative of the formula [1]. This combination of selecting the reaction solvent and controlling the amount of the reaction solvent makes it possible that the post-treatment operation can suitably be applied to the post-reaction solution even if the post-reaction solution results in a heterogeneous system where a part of the target compound has been precipitated.

The post-treatment operation according to one of the important features of the present invention will be explained in more detail below.

It suffices the amount of the water added is one fifth to five times the amount of the reaction solvent used. The amount of the water added is generally preferably in the range of one fourth to four times, more preferably one third to three times, the amount of the reaction solvent used. The preferred combination of "selecting the water-miscible organic solvent as the reaction solvent and forming and recovering the crystalline precipitate of the target compound with the addition of water to the post-reaction solution in the amount of one third to three times the amount of the reaction solvent" makes it possible to achieve a high salt removing effect and a high recovery rate of the target compound.

There is no particular limit on the crystalline precipitation technique. It is preferable to form the crystalline precipitate with stirring. It is particularly preferable to combine pulverization of the crystalline precipitate into the crystalline precipitation technique. The crystalline precipitate can be formed smoothly and efficiently with the use of a seed crystal as needed.

The amount of the seed crystal used is not particularly limited. It suffices to use 0.00001 mole or more of the seed crystal per 1 mole of the D-galactose derivative of the formula [1]. The amount of the seed crystal used is generally preferably in the range of 0.0001 1 to 0.1 mole, more preferably 0.0002 to 0.05 mole, per 1 mole of the D-galactose derivative of the formula [1].

It suffices that the precipitation temperature ranges from -20 to +50°C. The precipitation temperature is generally preferably in the range of -10 to +40°C, more preferably 0 to +30°C.

The precipitation time is not particularly limited. It suffices to form the precipitate in the range of 0.1 to 72 hours. The precipitation time depends on the target compound and the precipitation conditions. It is preferable determine the time at which almost all of the target compound has been precipitated as the end of the precipitation while monitoring the amount of the target compound in the supernatant liquor by any analytical means such as gas chromatography, liquid chromatography or NMR.

There is no particular limit on the recovery technique. In general, the 4-deoxy-4-fluoro-D-glucose derivative of the formula [2] can be recovered in crude crystalline form with almost no "salt of fluorosulfuric acid (or perfluoroalkanesulfonic acid) and organic base" by filtering the crystalline precipitate. The crude product or crude crystal of the target compound may be purified to a high chemical purity by purification operation such as activated carbon treatment, distillation or recrystallization as needed.

The recrystallization suitable as the purification operation will be explained in detail below.

Examples of the recrystallization solvent are: aliphatic hydrocarbon solvents such as n-pentane, n-hexane, cyclohexane and n-heptane; aromatic hydrocarbon solvents such as benzene, toluene, ethylbenzene, xylene and mesitylene; halogenated hydrocarbon solvents such as methylene chloride, chloroform and 1,2-dichloroethane; ether solvents such as diethyl ether, tetrahydrofuran, tert-butyl methyl ether and 1,4-dioxane; ketone solvents such as acetone, methyl ethyl ketone and methyl i-butyl ketone; ester solvents such as ethyl acetate and n-butyl acetate; nitrile solvents such as acetonitrile and propionitrile; alcohol solvents such as methanol, ethanol, n-propanol, i-propanol and n-butanol; and water. Among others, n-hexane, n-heptane, toluene, xylene, t-butyl methyl ether, acetone, ethyl acetate, acetonitrile, methanol, ethanol, n-propanol and i-propanol are preferred. Particularly preferred are n-hexane, n-heptane, toluene, xylene, ethyl acetate and i-propanol. These recrystallization solvents can be used solely or in any combination thereof.

The amount of the recrystallization solvent used is not particularly limited. It suffices to use 0.1 L or more of the recrystallization solvent per 1 mole of the crude product or crude crystal of the 4-deoxy-4-fluoro-D-glucose derivative of the formula [2]. The amount of the recrystallization solvent used is generally preferably in the range of 0.1 to 20 L, more preferably 0.1 to 10 L, per 1 mole of the crude product or crude crystal of the 4-deoxy-4-fluoro-D-glucose derivative of the formula [2].

In the recrystallization purification operation, the crystalline precipitate may be formed smoothly and efficiently with the addition of a seed crystal.

The amount of the seed crystal used is not particularly limited. It suffices to use 0.00001 mole or more of the seed crystal per 1 mole of the crude product or crude crystal of the 4-deoxy-4-fluoro-D-glucose derivative of the formula [2]. The amount of the seed crystal used is generally preferably in the range of 0.0001 to 0.1 mole, more preferably 0.0002 to 0.05 mole, per 1 mole of the crude product or crude crystal of the 4-deoxy-4-fluoro-D-glucose derivative of the formula [2].

The recrystallization temperature is not particularly limited and can be set as appropriate depending on the boiling point and freezing point of the recrystallization solvent used. In general, it is preferable to dissolve the unpurified target compound in the recrystallization solvent in the range of room temperature (25°C) to a temperature in the vicinity of the boiling point of the recrystallization solvent, and then, form the crystalline precipitate of the target compound in the range of -30 to +60°C.

As the recrystallization purification operation increases the chemical purity of the crystalline precipitate, the 4-deoxy-4-fluoro-D-glucose derivative of the formula [2] can be obtained with high chemical purity by recovering the crystalline precipitate by filtration. The target compound can be obtained with higher chemical purity by repeatedly performing the recrystallization purification operation. In the present invention, there occurs almost no impurity that is difficult to separate from the target compound. The target compound can be thus purified to be nearly pure (chemical purity 100%).

As described above, the 4-deoxy-4-fluoro-D-glucose derivative is produced by reacting the D-galactose derivative with sulfuryl fluoride, trifluoromethanesulfonyl fluoride or perfluorobutanesulfonyl fluoride in the presence of the organic base in the present invention (first embodiment).

Preferably, the 4-deoxy-4-fluoro-α-D-glucopyranoside derivative is produced by reacting the α-D-galactopyranoside derivative with sulfuryl fluoride or trifluoromethanesulfonyl fluoride in the presence of the organic base and forming and recovering the crystalline precipitate of the target compound with the addition of water to the post-reaction solution. The above production method uses as the raw material the α-D-galactopyranoside derivative, which is relatively easy to prepare, and maximize the merit of the post-treatment operation with the use of sulfuryl fluoride or trifluoromethanesulfonyl fluoride as the dehydroxyfluorination agent. This combination is thus a preferred embodiment (second embodiment) of the present invention.

More preferably, the methyl 2,3,6-tri-O-benzoyl-4-deoxy-4-fluoro-α-D-glucopyranoside is produced by reacting the methyl 2,3,6-tri-O-benzoyl-α-D-galactopyranoside with sulfuryl fluoride in the presence of triethylamine and forming and recovering the crystalline precipitate of the target compound with the addition of water to the post-reaction solution. The above production method enables direct production of the methyl 2,3,6-tri-O-benzoyl-4-deoxy-4-fluoro-α-D-glucopyranoside, which is a particularly important intermediate of drugs for diabetes, uses triethylamine available industrially at low cost and maximize the merit of the post-treatment operation with the use of sulfuryl fluoride as the dehydroxyfluorination agent. This combination is thus a particularly preferred embodiment (third embodiment) of the present invention.

The first embodiment can be made more effective by performing the reaction in the presence of "the salt or complex of the organic base and hydrogen fluoride" (fourth embodiment). The second embodiment can be made more effective by performing the reaction in the presence of "the salt or complex of the organic base and hydrogen fluoride" (fifth embodiment). The third embodiment can be made more effective by performing the reaction in the presence of "the salt or complex of triethylamine and hydrogen fluoride" (sixth embodiment).

In view of industrial applications, the second and third embodiments can be made industrially more practical by using the water-miscible organic solvent as the reaction solvent and forming and recovering the crystalline precipitate of the target compound with the addition of water to the post-reaction solution in the amount of one third to three times the amount of the reaction solvent (seventh and eighth embodiments); and the fifth and sixth embodiments can be made industrially easily feasible by using the water-miscible organic solvent as the reaction solvent and forming and recovering the crystalline precipitate of the target compound with the addition of water to the post-reaction solution in the amount of one third to three times the amount of the reaction solvent (ninth and tenth embodiments).

### [Examples]

The present invention will be described in more detail below by way of the following examples. It is however noted that these examples are not intended to limit the present invention thereto.

### [Example 1]

A pressure-proof reaction vessel of stainless steel (SUS) was charged with 400 g (790 mmol, 1.00 eq) of methyl 2,3,6-tri-O-benzoyl-α-D-galactopyranoside of the following formula, 2000 mL of acetonitrile, 200 g (1976 mmol, 2.50 eq) of triethylamine and 63.6 g (395 mmol, 0.50 eq) of triethylamine tris(hydrogen fluoride) complex, followed by lowering the inside temperature of the reaction vessel to -20°C and blowing 125 g (1225 mmol, 1.55 eq) of sulfuryl fluoride (SO₂F₂) from a cylinder into the reaction vessel under reduced pressure. 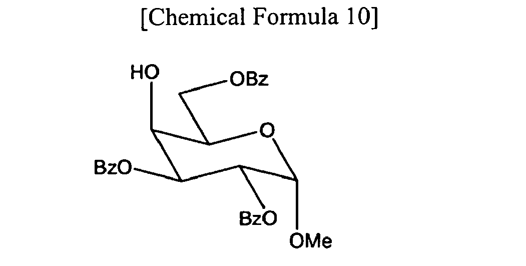 The resulting mixture was stirred over night at room temperature. The conversion rate of the reaction was determined by liquid chromatography to be 99.2%. The thus-obtained post-reaction solution was diluted with 1800 mL of ethyl acetate and washed with 1064 g of an aqueous potassium carbonate solution (prepared from 164 g (1187 mmol, 1.50 eq) of potassium carbonate and 900 mL of water), thereby separating into an organic layer and an aqueous layer. The organic layer was recovered. The aqueous layer was further subjected to extraction with 600 mL of ethyl acetate. The extract was combined into the organic layer. The organic layer was washed four times with 1200 mL of water until the amount of the salt of fluorosulfuric acid and triethylamine remaining in the organic layer became 1 mol% or less relative to the target compound. (The salt remaining amount after four times washing was determined by ¹⁹F-NMR to be 0.4 mol%.) The recovered organic layer was concentrated under reduced pressure and subjected to azeotropic dehydration with 1000 mL of toluene, thereby yielding 477 g of methyl 2,3,6-tri-O-benzoyl-4-deoxy-4-fluoro-α-D-glucopyranoside of the following formula as a crude crystal. 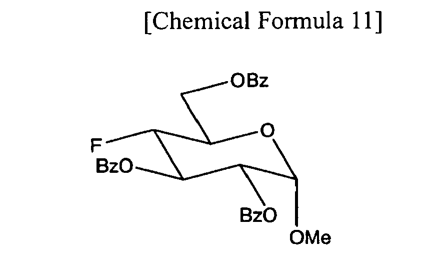 The purity of the crude crystal was determined by liquid chromatography to be 88.7% (4,5-olefin compound: 7.1%, chloro compound: 0.5%, unknown impurity: 1.8%). The whole of the crude crystal was admixed with 1200 mL of ethyl acetate and 1600 mL of n-heptane. The crude crystal was dissolved into the solvent by heating. The resulting solution was cooled to room temperature to form a crystalline precipitate. The crystalline precipitate was recovered by filtration, washed with 450 mL of n-heptane and vacuum dried, thereby yielding 330 g of a first recrystallized product. The purity of the first recrystallized product was determined by liquid chromatography to be 98.4% (4,5-olefin compound: 1.0%, chloro compound: 0.3%, unknown impurity: less than 0.1%). The total yield of the product from the reaction to the first recrystallization was 82%. The whole of the first recrystallized product was admixed with 990 mL of ethyl acetate and 1320 mL of n-heptane and dissolved in the solvent by heating. The resulting solution was cooled to room temperature to form a crystalline precipitate. The crystalline precipitate was recovered by filtration, washed with 400 mL of n-heptane and vacuum dried, thereby yielding 291 g of a second recrystallized product. The purity of the second recrystallized product was determined by liquid chromatography to be 99.6% (4,5-olefin compound: 0.1%, chloro compound: 0.2%, unknown impurity: less than 0.1 %). There was no salt of fluorosulfuric acid and triethylamine contained in the second recrystallized product. The total yield of the product from the reaction to the second recrystallization was 72%. The instrumental data of the obtained target compound are indicated below.

¹H-NMR (standard material: Me₄Si, deuterium solvent: CDCl₃), δ ppm: 3.47 (S, 3H), 4.32 (m, 1H), 4.67 (m, 2H), 4.76 (dt, 51.2Hz, 9.4Hz, 1H), 5.19 (m, 2H), 6.13 (dt, 14.4Hz, 9.6Hz, 1H), 7.34-7.64 (Ar-H, 9H), 7.95-8.13 (Ar-H, 6H).
¹⁹F-NMR (standard material: C₆F₆, deuterium solvent: CDCl₃), δ ppm: -35.32 (dd, 50.2Hz, 13.7Hz, 1F)

The above instrumental data was the same as that described in Experiment Part of Non-Patent Document 1.

### [Example 2]

A pressure-proof reaction vessel of stainless steel (SUS) was charged with 40.0 g (79.0 mmol, 1.00 eq) of methyl 2,3,6-tri-O-benzoyl-α-D-galactopyranoside of the following formula, 158 mL of acetonitrile, 20.0 g (198 mmol, 2.51 eq) of triethylamine and 6.36 g (39.5 mmol, 0.50 eq) of triethylamine tris(hydrogen fluoride) complex, followed by lowering the inside temperature of the reaction vessel to -5°C and blowing 18.3 g (179 mmol, 2.27 eq) of sulfuryl fluoride (SO₂F₂) from a cylinder into the reaction vessel under reduced pressure. 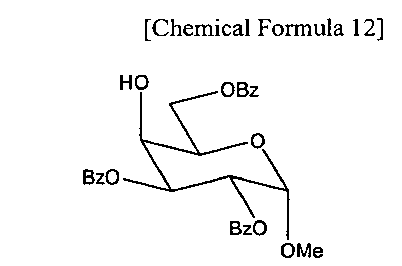 The resulting mixture was stirred for 2 hours at the same temperature as above. The mixture was further stirred over night at room temperature. The conversion rate of the reaction was determined by liquid chromatography to be 99.7%. The thus-obtained post-reaction solution was admixed with 237 mL of water and then stirred for 3 hours at room temperature to form a crystalline precipitate. The crystalline precipitate was filtered and washed with an aqueous acetonitrile solution (prepared from 20 mL of acetonitrile and 30 mL of water), thereby yielding 47.2 g of methyl
2,3,6-tri-O-benzoyl-4-deoxy-4-fluoro-α-D-glucopyranoside of the following formula as a crude crystal (wet cake). 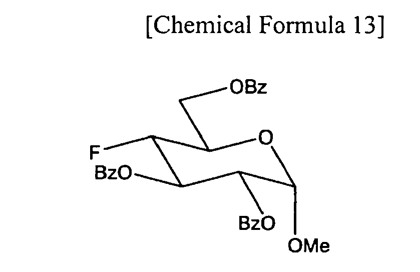 The purity of the crude crystal (wet cake) was determined by liquid chromatography to be 90.9% (4,5-olefin compound: 5.3%, chloro compound: 0.1%, unknown impurity: 2.6%). There was no salt of fluorosulfuric acid and triethylamine contained in the crude crystal (wet cake). (The salt remaining amount was determined by ¹⁹F-NMR.) The whole of the crude crystal (wet cake) was admixed with 120 mL of ethyl acetate and dissolved in the solvent by heating. The resulting solution was admixed with 160 mL of n-heptane and then cooled to room temperature to form a crystalline precipitate. The crystalline precipitate was recovered by filtration, washed with an ethylacetate/n-heptane mixed solution (prepared from 15 mL of ethyl acetate and 20 mL of n-heptane) and vacuum dried, thereby yielding 31.3 g of a first recrystallized product. The purity of the first recrystallized product (wet cake) was determined by liquid chromatography to be 98.9% (4,5-olefin compound: 0.9%, chloro compound: less than 0.1 %, unknown impurity: 0.1 %). The total yield of the product from the reaction to the first recrystallization was 78%. After that, 31.0 g of the first recrystallized product was admixed with 93 mL of ethyl acetate and 124 mL of n-heptane and dissolved in the solvent by heating. The resulting solution was cooled to room temperature to form a crystalline precipitate. The crystalline precipitate was recovered by filtration, washed with n-heptane and vacuum dried, thereby yielding 27.6 g of a second recrystallized product. The purity of the second recrystallized product (wet cake) was determined by liquid chromatography to be 99.8% (4,5-olefin compound: 0.1 %, chloro compound: less than 0.1%, unknown impurity: less than 0.1 %). The total yield of the product from the reaction to the second recrystallization was 69%. The instrumental data of the obtained target compound was the same as that of Example 1.

In this way, the post-treatment operation of Example 2 was much more advantageous in view of all aspects such as operability, waste amount and salt removal efficiency as summarized in TABLE 1 although the reaction proceeds favorably in both of Example 1 and Example 2.

**TABLE 1**

| Example | 1 | 2 |
|---|---|---|
| Salt removal | washing four times with water → concentration | precipitation→ filtration |
| Raw material | 1 weight | 1 weight |
| Waste organic solvent | 11 volumes | 4 volumes |
| Waste water | 14 volumes | 7 volumes |
| Salt remaining amount | 0.4 mol% | undetectable |

### [Example 3]

A pressure-proof reaction vessel of stainless steel (SUS) was charged with 507 mg (1.001 mmol, 1.00eq) of methyl 2,3,6-tri-O-benzoyl-α-D-galactopyranoside of the following formula, 1 mL of acetonitril and 202 mg (1.996 mmol, 1.99eq) of triethylamine, followed by lowering the inside temperature of the reaction vessel to -78°C and blowing 304 mg (1.999 mmol, 2.00eq) of trifluoromethanesulfonyl fluoride (CF₃SO₂F) from a cylinder into the reaction vessel. 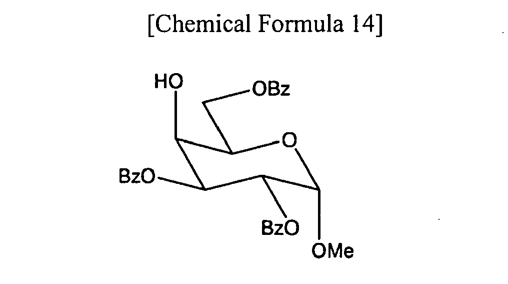 The resulting mixture was stirred over night at room temperature. The thus-obtained post-reaction solution was diluted with ethyl acetate and washed with an aqueous solution of potassium carbonate. The organic layer was recovered and concentrated under reduced pressure, thereby yielding 610 mg of methyl
2,3,6-tri-O-benzoyl-4-deoxy-4-fluoro-α-D-glucopyranoside of the following formula as a crude product. 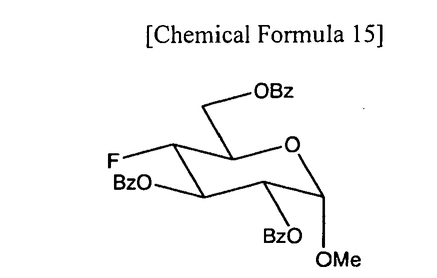 The crude product was verified by ¹H-NMR and ¹⁹F-NMR to contain the target compound as a main product (according to comparison with the instrumental date of Example 1).

### [Example 4]

A pressure-proof reaction vessel of stainless steel (SUS) was charged with 300 mg (0.592 mmol, 100eq) of methyl 2,3,6-tri-O-benzoyl-α-D-galactopyranoside of the following formula, 0.6 mL of acetonitrile, 240 mg (2.372 mmol, 4.01eq) of triethylamine and 95.5 mg (0.592 mmol, 1.00eq) of triethylamine tris(hydrogen fluoride) complex, followed by lowering the inside temperature of the reaction vessel to -78°C and blowing 2090 mg (13.744 mmol, 23.22eq) of trifluoromethanesulfonyl fluoride (CF₃SO₂F) from a cylinder into the reaction vessel. 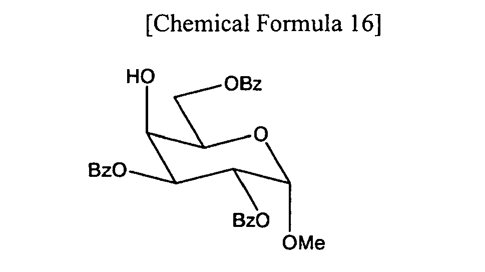 The resulting mixture was stirred over night at room temperature. The thus-obtained post-reaction solution was diluted with ethyl acetate and washed with an aqueous solution of potassium carbonate. The organic layer was recovered and concentrated under reduced pressure, thereby yielding 492 mg of methyl
2,3,6-tri-O-benzoyl-4-deoxy-4-fluoro-α-D-glucopyranoside of the following formula as a crude product. 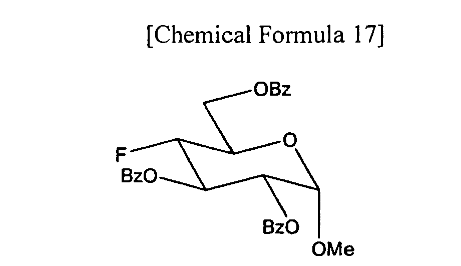 The crude product was verified by ¹H-NMR and ¹⁹F-NMR to contain the target compound as a main product (according to comparison with the instrumental date of Example 1).

## Claims

1. A production method of a 4-deoxy-4-fluoro-D-glucose derivative of the formula [2], comprising:
performing a reaction of a D-galactose derivative of the formula [1] with either sulfuryl fluoride (SO₂F₂), trifluoromethanesulfonyl fluoride (CF₃SO₂F) or perfluorobutanesulfonyl fluoride (C₄F₉SO₂F) in the presence of an organic base 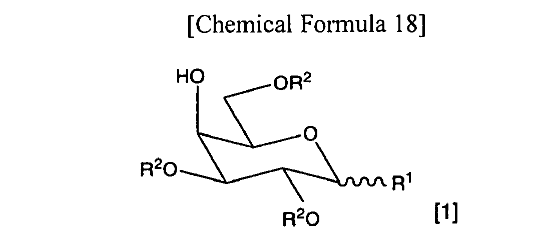 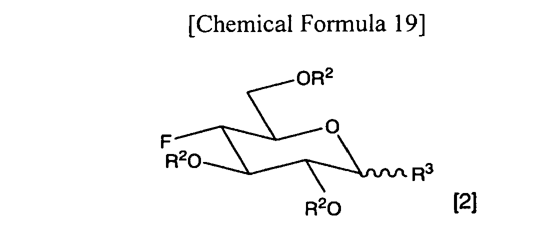 where R¹ represents a hydroxyl group, a lower alkoxy group, a lower acyloxy group or a halogen atom; R² independently represents a hydroxyl protecting group; R³ represents a lower alkoxy group, a lower acyloxy group or a halogen atom; and the wavy line represents the presence of either or both of α-anomer configuration and β-anomer configuration.

2. The production method of the 4-deoxy-4-fluoro-D-glucose derivative according to claim 1, wherein the reaction is performed in the additional presence of a salt or complex of an organic base and hydrogen fluoride.

3. A production method of a 4-deoxy-4-fluoro-α-D-glucopyranoside derivative of the formula [4], comprising:
performing a reaction of an α-D-galactopyranoside derivative of the formula [3] with either sulfuryl fluoride(SO₂F₂) or trifluoromethanesulfonyl fluoride (CF₃SO₂F) in the presence of an organic base, thereby obtaining a post-reaction solution containing the 4-deoxy-4-fluoro-α-D-glucopyranoside derivative as a target compound; and
forming and recovering a crystalline precipitate of the target compound with the
addition of water to the post-reaction solution 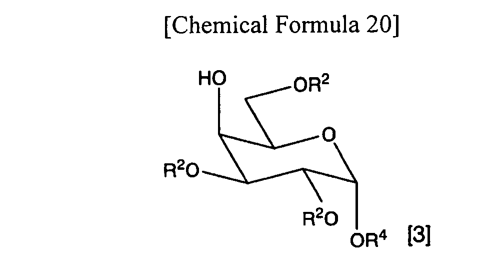 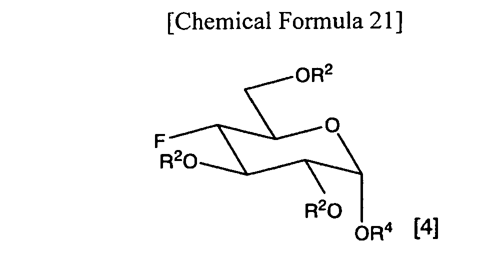 where R² independently represents a hydroxyl protecting group; and R⁴ represents a lower alkyl group.

4. The production method of the 4-deoxy-4-fluoro-α-D-glucopyranoside derivative according to claim 3, wherein the reaction is performed in the additional presence of a salt or complex of an organic base and hydrogen fluoride.

5. The production method of the 4-deoxy-4-fluoro-α-D-glucopyranoside derivative according to claim 3 or 4, wherein a water-miscible organic solvent is used as a solvent of the reaction; and an amount of the water added to the post-reaction solution to form and recover the crystalline precipitate of the target compound is one third to three times an amount of the solvent of the reaction.

6. A production method of methyl
2,3,6-tri-O-benzoyl-4-deoxy-4-fluoro-α-D-glucopyranoside of the formula [6], comprising:
performing a reaction of methyl 2,3,6-tri-O-benzoyl-α-D-galactopyranoside of the formula [5] with sulfuryl fluoride (SO₂F₂) in the presence of triethylamine, thereby obtaining a post-reaction solution containing the methyl
2,3,6-tri-O-benzoyl-4-deoxy-4-fluoro-α-D-glucopyranoside as a target compound; and
forming and recovering a crystalline precipitate of the target compound with the
addition of water to the post-reaction solution 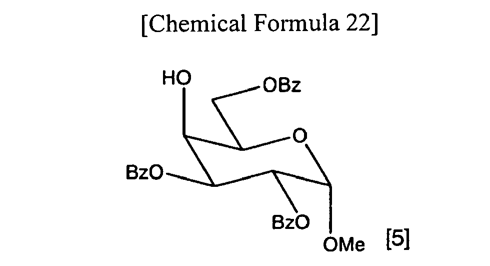 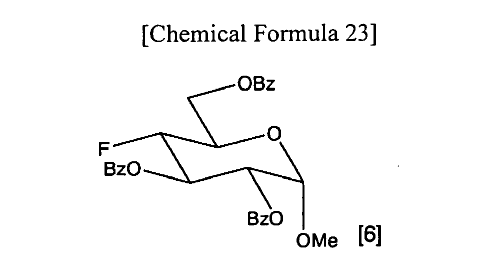 where Me represents a methyl group; and Bz represents a benzoyl group.

7. The production method of the methyl 2,3,6-tri-O-benzoyl-4-deoxy-4-fluoro-α-D-glucopyranoside according to claim 6, wherein the reaction is performed in the additional presence of a salt or complex of triethylamine and hydrogen fluoride.

8. The production method of the methyl 2,3,6-tri-O-benzoyl-4-deoxy-4-fluoro-α-D-glucopyranoside according to claim 6 or 7, wherein a water-miscible organic solvent is used as a solvent of the reaction; and an amount of the water added to the post-reaction solution to form and recover the crystalline precipitate of the target compound is one third to three times an amount of the solvent of the reaction.
